# EUROPÄISCHE PATENTANMELDUNG

(11) **EP 1 362 552 A1**
(43) Veröffentlichungstag der Anmeldung: **19.11.2003**
(21) Anmeldenummer: 03010333.7
(22) Anmeldetag: 08.05.2003
(51) Int. Cl.: A61B 5/22, G01L 1/22, G01L 3/24, G01L 5/16, A63B 22/08, B62J 39/00

(54) **Verfahren und Vorrichtung zur Auswertung der auf eine Tretkurbel ausgeübten Kräfte**

(30) Priorität: 14.05.2002 DE 10221519
(71) Anmelder: GTM GASSMANN THEISS MESSTECHNIK GMBH, 64404 Bickenbach (DE)
(72) Erfinder: Schwind, Daniel, 55234 Wendelsheim (DE)
(74) Vertreter: Katscher, Helmut, Dipl.-Ing.

(57) **Zusammenfassung**

Bei einem Verfahren zur Auswertung der auf eine Tretkurbel eines Fahrrads oder Ergometers ausgeübten, gemessenen Kräfte werden die nicht in tangentialer Richtung im Umdrehungssinn an der Tretkurbel angreifenden Kräfte ermittelt und akustisch signalisiert. Eine betragsmäßige Änderung der ermittelten Kräfte wird durch eine damit korrelierende Frequenzänderung des akustischen Signals angezeigt. Eine Vorrichtung zur Durchführung des Verfahrens mit einer Kraftmessvorrichtung einer damit in Verbindung stehenden Auswertevorrichtung ist in einem Pedal (1) angeordnet. Mittels Dehnungsmessstreifen (7) werden in tangentialer und radialer Richtung auf eine Tretkurbel wirkende Kräfte gemessen und nach Verstärkung und Aufbereitung der Messsignale zur Ansteuerung eines Lautsprechers (11) verwendet. Durch die Anordnung der Dehnungsmessstreifen (7) sowie die verwendete Verstärkerelektronik (10) wird erreicht, dass der gemessene Anteil der nicht in tangentialer Richtung im Umdrehungssinn an der Tretkurbel angreifenden Kräfte akustisch angezeigt wird. Die Auswertevorrichtung kann eine Einschaltautomatik sowie einen Speicher zum Abspeichern der gemessenen Signale aufweisen.

## Beschreibung

Die Erfindung betrifft ein Verfahren zur Auswertung der auf eine Tretkurbel eines Fahrrads oder Ergometers ausgeübten, gemessenen Kräfte.

In der Radsport-Trainingslehre hat sich zur Optimierung des Pedalierens seit Jahrzehnten der Begriff des runden Tritts etabliert, der als Maß für die Gleichmäßigkeit der vom Radsportler auf die Tretkurbel übertragenen Kraft verwendet wird. Dabei wird nur der tangentiale Anteil der im Umdrehungssinn über die Pedale auf die Tretkurbel übertragenen Kräfte in Bewegungsenergie umgesetzt. Nicht tangential angreifende Kräfte oder tangentiale Kräfte entgegen dem Umdrehungssinn der Tretkurbel werden nicht in Bewegungsenergie umgesetzt oder verringern sogar die in Bewegungsenergie umgesetzte Leistung.

Bei Pedalen ohne Haltevorrichtung kann offensichtlich nur während etwa einer halben Umdrehung der Tretkurbel über ein Pedal tangentiale Kraft in Umdrehungsrichtung auf die Tretkurbel übertragen werden. Der Tangentialanteil der übertragenen Kräfte ist dabei bei etwa waagrechter Stellung der Tretkurbelarme am größten und verschwindend gering bei senkrechter Stellung der Tretkurbelarme. Bei einem untrainierten Radler wird üblicherweise die durch das abwärts bewegte Bein auf die Tretkurbel übertragene Kraft nur zum Teil in Bewegungsenergie umgesetzt, da das andere, auf dem zweiten Pedal ruhende Bein die aufwärts gerichtete Drehbewegung des zweiten Pedals hemmt. Durch Trainieren eines optimierten Bewegungsablaufs können die nicht in Bewegungsenergie umgesetzten Kräfte reduziert werden; ein runder Tritt ist jedoch prinzipiell nicht möglich.

Aus diesem Grund gibt es Systeme, bei denen sich mittels einer rastenden Bindung ein Radfahrerschuh formschlüssig mit einem daran angepassten Pedal verbinden lässt. Auf diese Weise können bei beliebiger Stellung der Tretkurbel Kräfte auf diese ausgeübt werden. Damit wird einerseits eine gleichmäßigere Auslastung des Radfahrers und andererseits das Einbeziehen weiterer, leistungsfähiger Muskelgruppen während des Radfahrens ermöglicht. Derartige Pedalbindungen finden breite Anwendung im Hobby-, Amateurund Profibereich.

Es sind Verfahren und Vorrichtungen bekannt, mittels derer bei einem Ergometer die auf die Tretkurbel übertragenen Kräfte, bzw. die in Bewegungsenergie umsetzbare Leistung gemessen werden kann. Zur Verbesserung der Bewegungsabläufe wird der runde Tritt durch intensives Trainieren unter Verwendung solcher Ergometer und zeitnaher Auswertung und Beurteilung der gemessenen Kräfte oder Leistungen gezielt trainiert und verbessert.

Es sind auch Verfahren und Vorrichtungen bekannt und beispielsweise in der Patentschrift DE 37 22 728 C1 beschrieben, bei denen eine Kraft- und/oder Leistungsmessung auf einem unter realistischen Bedingungen eingesetzten Trainingsfahrrad erfolgen kann. Dabei werden meistens dafür vorgesehene Tretkurbeln oder Pedale mit daran angeordneten Kraftmessvorrichtungen und einer daran angeschlossenen Auswerteeinheit verwendet. Die gemessenen Kräfte werden üblicherweise in radiale und tangentiale Anteile zerlegt und die tangentialen Kräfte auf einem Display angezeigt und/oder zur späteren Analyse abgespeichert.

Bei derartigen Verfahren erhält der Sportler eine ständige optische Rückmeldung, wie groß der Anteil der in Bewegung umsetzbaren Energie ist, der durch die auf die Tretkurbel ausgeübten Kräfte bei seinem Bewegungsablauf umgesetzt wird. Nach jüngeren Ergebnissen der psychologischen Lernforschung ist diese Trainingsmethode jedoch von mangelnder Effizienz, weil der Sportler keine Möglichkeit hat, die Qualität des ausgeübten Bewegungsablaufs selbst beurteilen zu können. So kann der Trainierende die in Bewegung umsetzbare Energie in gewissem Maße durch erhöhte Leistung steigern, er kann bei diesem Verfahren aber nicht einen verbesserten Bewegungsablauf und einen optimierten runden Tritt erlernen, so dass ein Teil der durch den Sportler erbrachten Leistung nicht in Bewegungsenergie umsetzbar ist und zu einer schnelleren Ermüdung des Sportlers führt.

Aufgabe der Erfindung ist es demzufolge, ein Verfahren zur Auswertung der auf eine Tretkurbel eines Fahrrads oder Ergometers ausgeübten, gemessenen Kräfte so zu gestalten, dass der Trainierende jederzeit die Qualität seiner ausgeübten Bewegungsabläufe kontrollieren und beurteilen kann. Das Verfahren soll mit einfachen Mitteln bei Fahrrädern und Ergometern anwendbar sein und die Aufmerksamkeit und Konzentration des Trainierenden nicht unnötig stören.

Diese Aufgabe wird erfindungsgemäß durch ein Verfahren gelöst, bei dem die nicht in tangentialer Richtung im Umdrehungssinn angreifenden Kräfte ermittelt und akustisch signalisiert werden.

Während bei bekannten Verfahren regelmäßig die Kraft- oder Leistungsanteile ermittelt und angezeigt werden, die in Bewegungsenergie umsetzbar sind, werden bei dem erfindungsgemäßen Verfahren gerade die nicht in Bewegungsenergie umsetzbaren Kräfte ermittelt und angezeigt, so dass der Trainierende gezielt darauf hingewiesen wird, dass ein Teil der von ihm erbrachten Leistung nutzlos ist. Zum Erlernen einer ermüdungsarmen Tritttechnik ist dieses Verfahren, welches die Aufmerksamkeit des Trainierenden auf unerwünschte Effekte seiner Bewegungsabläufe und nicht auf den Bewegungsablauf selbst lenken, besonders geeignet.

Eine ständige Kontrolle einer optischen Anzeige durch den Trainierenden selbst ist wenig geeignet, da seine Konzentration und auch Körperhaltung auf die Wahrnehmung der optischen Anzeige gerichtet sind. Während dies bei Ergometern noch möglich ist, dürfte die Verwendung einer optischen Anzeige im Straßenverkehr kaum sinnvoll für eine kontinuierliche Überwachung und Rückmeldung des Bewegungsablaufs geeignet sein. Eine nachträgliche Aufarbeitung und optische Darstellung beispielsweise einer gesamten Trainingseinheit kann sinnvoll sein, ermöglicht dem Trainierenden jedoch nicht die unmittelbare Kontrolle seines Bewegungsablaufs und gegebenenfalls eine sofortige Korrektur desselben.

Aus diesem Grund werden die ermittelten Kräfte akustisch signalisiert, so dass das Verfahren auch mit geringst möglicher Beeinträchtigung im Straßenverkehr verwendet werden kann. Dadurch ist ein Trainieren auch unter realistischen Bedingungen möglich.

Vorzugsweise ist vorgesehen, dass eine betragsmäßige Änderung der ermittelten Kräfte durch eine vorbestimmte Frequenzänderung des akustischen Signals angezeigt wird. Auf diese Weise ist es möglich, nicht nur das Auftreten unerwünschter Kräfte während des Bewegungsablaufs zu signalisieren, sondern auch eine Rückmeldung über deren betragsmäßigen Anteil zu geben. So kann der Trainierende anhand der einfach wahrnehmbaren und gut unterscheidbaren Signalfrequenz überprüfen, ob im Verlauf des Trainings eine Verbesserung des Bewegungsablaufs erfolgt, auch wenn immer noch unerwünschte Kräfte auf die Tretkurbel übertragen werden.

Einer Ausgestaltung des Erfindungsgedankens zufolge ist vorgesehen, dass auch die in tangentialer Richtung im Umdrehungssinn angreifenden Kräfte und die Umdrehungsfrequenz ermittelt werden. Damit wird zusätzlich eine Auswertung der in Bewegungsenergie umsetzbaren Leistung möglich, so dass neben einer Verbesserung des Bewegungsablaufs auch eine Steigerung der Leistung durch den Trainierenden verfolgt werden kann.

Vorteilhafterweise ist vorgesehen, dass aus den ermittelten Kräften die dafür aufgewandte Leistung bestimmt und bei vorgebbaren Grenzwerten der Leistung ein akustisches Signal erzeugt wird. Gerade bei längeren Trainingseinheiten ist es wünschenswert, dass die von dem Trainierenden erbrachte Leistung sich innerhalb an seinen Leistungsstand angepasster Grenzwerte bewegt. Dadurch wird zum einen eine dauerhaft erbrachte, gleichmäßige Leistung des Trainierenden gefördert und eine nur kurzfristig mögliche Leistungssteigerung in Bereiche vermieden, die sportphysiologischen Erkenntnissen zufolge einem kontinuierlichen, dauerhaften Leistungsaufbau wenig förderlich wäre. Auf diese Weise können auch besondere Trainingssituationen wie beispielsweise Zeitfahren oder Erholungsphasen gezielt vorgegeben und trainiert werden.

Gemäß einer Ausgestaltung des Erfindungsgedankens ist vorgesehen, dass die Messwerte der ermittelten Kräfte und/oder bestimmten Leistungen in einem Speichermedium gespeichert werden. Nach Abschluss einer Trainingseinheit können die gespeicherten Messwerte abgerufen und beliebig ausgewertet und aufbereitet werden. So können auch komplexere Auswertungen vorgenommen und zur Optimierung des Bewegungsablaufs und der Steigerung der Leistungsfähigkeit verwendet werden.

Die Erfindung betrifft auch eine Vorrichtung zur Durchführung des erfindungsgemäßen Verfahrens mit einer Kraftmessvorrichtung und einer damit in Verbindung stehenden Auswertevorrichtung.

Es sind verschiedene Vorrichtungen zur Leistungsmessung bei Fahrrädern und Ergometern bekannt. Dabei wird regelmäßig eine auf die Tretkurbel übertragene Kraft gemessen und beispielsweise am Lenker optisch angezeigt. Oftmals werden auch weitere Kenngrößen wie beispielsweise die Umdrehungsfrequenz oder Winkelgeschwindigkeit der Tretkurbel gemessen und angezeigt bzw. für die Berechnung abgeleiteter Kenngrößen verwendet.

Die Patentschrift DE 37 22 728 C1 zeigt eine Vorrichtung zur Leistungsmessung, bei der die an der Kurbel angreifende Kraft über ein Verformungselement auf die Antriebsscheibe übertragen wird, mittels Dehnungsmessstreifen die gemessene Verformung in elektrische Signale umgewandelt wird und die Messwerte optisch angezeigt werden. Durch Ausgestaltung des Verformungselements und die Anordnung der Dehnungsmessstreifen vorgegeben, werden hierbei im wesentlichen radial angreifende Kräfte gemessen und zur Berechnung der aufgebrachten Leistung verwendet. Nachteilig ist zudem, dass die bisher verwendete Tretkurbel vollständig gegen eine Tretkurbel mit einem speziellen Verformungselement ausgetauscht werden muss, wobei die unterschiedlichsten Vorgaben der verschiedenen Hersteller für das jeweilige Tretkurbelgehäuse eine entsprechende Anzahl daran angepasster Tretkurbeln mit Verformungselement erforderlich machen.

In der Offenlegungsschrift DE 42 27 586 A1 wird ein Ergometer beschrieben, bei dem mittels Dehnungsmessstreifen Verformungen der Tretkurbelarme, des Lenkers und der Sattelstange kontinuierlich gemessen und optisch angezeigt werden. Eine derartige Vorrichtung ist aufwendig und kostenintensiv. Es ist eine verhältnismäßig komplexe Auswertung der gemessenen Verformungen notwendig, um die an der Tretkurbel radial angreifenden Kraftanteile zu ermitteln und anzuzeigen.

Bei einer bekannten Vorrichtung der eingangs genannten Gattung (DE 44 35 174 A1) wird mittels Dehnungsmessstreifen die Schubverformung an einem Kurbelzapfen gemessen und einem Auswertegerät zugeführt. Auch hier wird die der angreifenden Kraft zugehörige Winkelgeschwindigkeit und/oder der Kurbelwinkel aus einer Zeitmessung eines mit der Kurbeldrehung periodisch wirkenden Signals ermittelt und zur Berechnung der aufgewandten Leistung verwendet. Die auch hier notwendige Messung der Winkelgeschwindigkeit ist aufwendig und mit zusätzlichen Kosten verbunden. Um die optisch anzuzeigenden Kräfte, bzw. Leistungen zutreffend berechnen zu können, muss die Vorrichtung nach ihrer Montage aufwendig kalibriert werden.

Aufgabe der Erfindung ist es demzufolge auch, eine Vorrichtung zur Durchführung des Verfahrens so zu gestalten, dass das Verfahren mit einfachen Mitteln und möglichst geringem Montageaufwand bei Fahrrädern und Ergometern durchgeführt werden kann und auch unter den üblichen Bedingungen im Straßenverkehr eingesetzt werden kann, ohne die Sicherheit des Anwenders zu gefährden.

Diese Aufgabe wird erfindungsgemäß durch eine Vorrichtung gelöst, bei der mittels der Auswertevorrichtung der Anteil der nicht in tangentialer Richtung im Umdrehungssinn angreifenden Kräfte bestimmbar und ein davon abhängiges Signal als Steuerungssignal für einen akustischen Signalgeber verwendbar ist.

Ein akustischer Signalgeber, beispielsweise ein batteriebetriebener Lautsprecher, und dessen elektronische Ansteuerung kann aus handelsüblichen Bauteilen kostengünstig hergestellt und direkt am Tretkurbelarm oder an einem damit verbundenen Pedal angeordnet werden, so dass eine aufwendige Signalübermittlung zu einem teuren, optischen Anzeigedisplay entfällt. Die Art der Kraftmessung ist an unterschiedliche Anforderungen anpassbar und kann beispielsweise durch Dehnungsmessstreifen, Piezoelemente oder optische, bzw. magnetische Sensoren erfolgen. Da durch das akustische Signal nur das Auftreten, nicht aber der absolute Betrag der nicht tangential im Umdrehungssinn angreifenden Kräfte angezeigt wird, entfällt eine umständliche und aufwendige Kalibration der Vorrichtung.

Einer Ausgestaltung des Erfindungsgedankens zufolge ist vorgesehen, dass die Kraftmessvorrichtung an einer Pedalachse angeordnet ist. Sowohl die verwendete Kraftmessvorrichtung als auch der akustische Signalgeber können ohne weiteres in bzw. an einem Pedal angeordnet werden, welches zu Trainingszwecken das bislang verwendete Pedal ersetzen kann. Auf diese Weise können die Herstellungskosten sowie die notwendigen Umbaumaßnahmen gering gehalten werden. Für den Trainierenden ist es ohne Schwierigkeiten möglich, zwischen Signaltönen des rechten und linken Pedals zu unterscheiden und seine Bewegungen entsprechend zu korrigieren.

Vorzugsweise ist vorgesehen, dass die angreifenden Kräfte mittels Dehnungsmessstreifen ermittelbar sind. Die geeignete Anbringung und genaue Auswertung von Verformungsmessungen mittels Dehnungsmessstreifen ist seit langem bekannt und eine vielfach für die verschiedensten Anwendungen eingesetzte Technik. Dehnungsmessstreifen sind vorgefertigt kostengünstig erhältlich und können mit einfachen Mitteln auf den Bereichen angeordnet werden, deren Verformung gemessen werden soll. Bei einer vorteilhaften Anordnung bilden Dehnungsmessstreifen insgesamt zwei Wheatstone-Brücken, mit denen das Messen der Kräfte in tangentialer und radialer Richtung möglich ist. Es werden zweckmäßigerweise für eine Biegespannungsmessung ausgelegte Dehnungsmessstreifen verwendet, da diese zu den meist genutzten Dehnungsmessstreifen gehören und somit kostengünstig und in vielfältigen Ausführungsformen erhältlich sind.

Damit die Dehnungsmessstreifen tatsächlich jeweils ausschließlich in tangentiale und radiale Richtung Kräfte messen, muss die Achse des Pedals genau ausgerichtet montiert werden. Dies kann beispielsweise dadurch erreicht werden, dass die Pedalachse lagegerecht in die dafür vorgesehenen Bohrungen der Tretkurbelarme eingeschraubt und dann mittels einer Kontermutter fixiert wird.

Die radial angreifenden Kräfte, die ja nicht in Bewegungsenergie umgesetzt werden können, werden akustisch signalisiert, und zwar unabhängig davon, ob eine Zugkraft oder eine Druckkraft ausgeübt wird. Derartige Kräfte treten üblicherweise in den Abwärtsphasen des Pedals auf, wenn die Kurbelarme nicht genau waagrecht stehen und der Fuß beim Abwärtstreten nicht in idealer Weise geneigt wird.

Tangential angreifende Kräfte werden nur dann akustisch signalisiert, wenn sie entgegen der Umdrehungsrichtung angreifen. Dies tritt vor allem bei der Aufwärtsbewegung des Pedals auf, wenn die Gewichtskraft des aufwärts bewegten Beins auf das Pedal wirkt.

Vorteilhafterweise ist vorgesehen, dass die Abhängigkeit der erzeugten akustischen Signale von den gemessenen Kräften einstellbar ist. Je nach den Kenntnissen und dem aktuellen Trainingsstand zu Beginn der Nutzung einer derartigen Vorrichtung werden die Trainierenden unterschiedliche Bewegungsabläufe durchführen, so dass an das jeweilige Vermögen des Trainierenden angepasste Korrekturhinweise sinnvoll sind. Aus diesem Grund ist es möglich, die Empfindlichkeit der akustischen Anzeige einzustellen. Ein ungeübter Radfahrer wird sein Training zunächst mit einer unempfindlichen Einstellung beginnen, bis er seinen Bewegungsablauf so weit verbessert hat, dass keine akustische Signalisierung mehr erfolgt. Sodann kann er die Empfindlichkeit erhöhen, so dass bereits geringere Kräfte, die nicht in Bewegungsenergie umgesetzt werden können, akustisch angezeigt werden.

Wichtig für den Lernerfolg ist nicht die genaue betragsmäßige Kenntnis der unerwünschten Kräfte, sondern ob derartige Kräfte überhaupt auftreten, bzw. je nach Trainingsstand ab einem vorgebbaren Schwellenwert akustisch signalisiert werden. Für diesen Zweck ist eine unkalibrierte Vorrichtung verwendbar, die demzufolge auch von einem messtechnischen Laien montiert und in Betrieb genommen werden kann.

In einer einfachen Ausführung ist es nicht notwendig, die gemessenen Kräfte mit einer hohen Auflösung zu verstärken und beispielsweise für eine optische Anzeige aufzubereiten. Dies vereinfacht die Auswahl der Messverstärker und dient einer wesentlichen Kostenersparnis.

Einer Ausgestaltung des Erfindungsgedankens zufolge ist vorgesehen, dass eine relative Änderung der gemessenen Kräfte durch eine vorbestimmte Frequenzänderung des akustischen Signals anzeigbar ist. Bei einer derartigen Ausführungsform wird auch der Betrag der nicht in Bewegungsenergie umsetzbaren Kräfte ermittelt und in Abhängigkeit davon ein höherer oder ein tieferer Ton erzeugt. Diese Relation des ermittelten Betrags der Kraft mit der Frequenzänderung hat gegenüber einer entsprechenden Lautstärkeänderung Vorteile hinsichtlich der einfacheren Wahrnehmung auch bei im Straßenverkehr unvermeidlichen Störgeräuschen. Auch können Frequenzänderungen in der Regel besser erkannt und unterschieden werden als Lautstärkeänderungen.

Vorteilhafterweise ist vorgesehen, dass die akustischen Signale mittels mit der Auswertevorrichtung in Verbindung stehenden Kopf- oder Ohrhörern erzeugbar sind. Dies ist insbesondere dann von Vorteil, wenn durch ähnliche oder sehr laute Geräusche die akustischen Signale der Vorrichtung nur noch schwer oder undeutlich wahrnehmbar sind. Die Steuersignale können dabei beispielsweise mittels einer Funkübertragung von der Auswertevorrichtung an daran angepasste Kopf- oder Ohrhörer übertragen werden.

Vorzugsweise ist vorgesehen, dass an dem Pedal ein Umdrehungssensor und ein Energiespeicher angeordnet sind, die jeweils mit der Auswertevorrichtung in Verbindung stehen. Bei einer derartigen Vorrichtung kann die Verstärkerelektronik der Auswertevorrichtung dadurch eingeschaltet werden, dass beispielsweise mit einem Magnet und einem zugeordneten Sensor jede Pedalumdrehung gemessen wird und einen entsprechenden elektrischen Impuls erzeugt. Damit kann erreicht werden, dass die Verstärkerelektronik automatisch aktiviert wird, sobald der Radfahrer mit dem Training beginnt. Der Energiespeicher, beispielsweise eine handelsübliche Batterie oder ein wieder aufladbarer Akkumulator, versorgt die Auswertevorrichtung und die daran angeschlossenen Sensoren mit Energie. Es ist auch möglich und oftmals sinnvoll, den zur akustischen Signalerzeugung verwendeten Lautsprecher ebenfalls durch den Energiespeicher zu versorgen.

Gemäß einer Ausgestaltung des Erfindungsgedankens ist vorgesehen, dass auch die in tangentialer Richtung im Umdrehungssinn angreifenden Kräfte und die Umdrehungsfrequenz ermittelbar sind. Damit wird die Bestimmung der in Bewegungsenergie umsetzbaren Kräfte, bzw. Leistung ermöglicht. Der Trainierende kann dann sowohl seinen Bewegungsablauf optimieren als auch seine Leistungssteigerung kontrollieren.

Vorteilhafterweise ist vorgesehen, dass bei vorgebbaren Grenzwerten der gemessenen Leistung ein akustisches Signal erzeugbar ist. Mittels einstellbarer unterer Grenzwerte lässt sich beispielsweise feststellen, wann die vom Trainierenden erbrachte Leistung unter ein gewünschtes Niveau abfällt, um dann ein akustisches Signal zu erzeugen. Diese Funktion ist insbesondere beim Zeitfahrtraining sehr hilfreich. Bei Überschreiten der Leistung eines oberen Grenzwertes kann ebenfalls ein akustisches Signal erzeugt werden. Dieser Hinweis kann insbesondere für Fahrten während gewünschter Erholungsphasen von Bedeutung sein. Weiterhin kann der obere Grenzwert als Warneinrichtung für orthopädische Zwecke genutzt werden, wenn beispielsweise das Kniegelenk nicht übermäßig belastet werden soll.

Einer Ausgestaltung des Erfindungsgedankens zufolge ist vorgesehen, dass die Messwerte der gemessenen Kräfte und/oder Leistung in einem Speichermedium speicherbar sind. Im Anschluss an eine Trainingseinheit können die gespeicherten Messwerte ausgelesen und zu einer komplexen Auswertung beispielsweise an einen Computer übertragen werden. Auch ist es möglich, durch einen Langzeitvergleich der gespeicherten Messwerte die Entwicklung über einen längeren Zeitraum zu dokumentieren und analysieren.

Weitere Ausgestaltungen des Erfindungsgedankens sind Gegenstand weiterer Unteransprüche.

Nachfolgend werden Ausführungsbeispiele näher erläutert, die in der Zeichnung dargestellt sind. Es zeigt:
Fig. 1 eine geschnittene Ansicht eines Pedals mit einer Kraftmessvorrichtung und einer damit in Verbindung stehenden Auswertevorrichtung,
Fig. 2 einen schematischen Aufbau der Auswertevorrichtung mit einem daran angeschlossenen Lautsprecher und
Fig. 3 einen schematischen Aufbau einer anderen Auswertevorrichtung mit einer Einschaltautomatik.

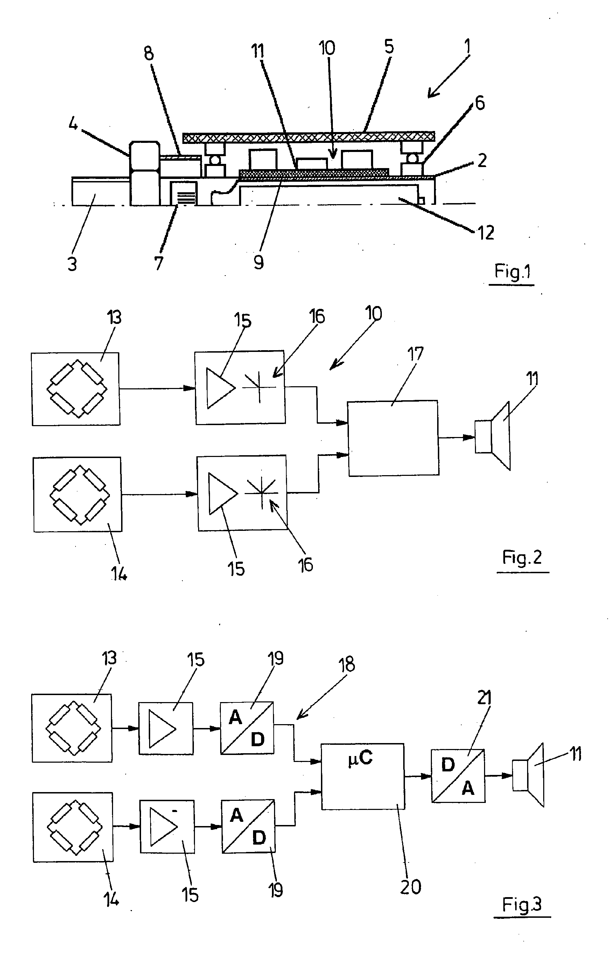

In Fig. 1 ist ein Pedal 1 mit einer Hohlachse 2 dargestellt, bei dem die Hohlachse 2 mittels eines Gewindezapfens 3 starr mit einem nicht dargestellten Kurbelarm verbunden werden kann. Die Ausrichtung der Hohlachse 2 relativ zu dem Kurbelarm kann mittels einer Kontermutter 4 fixiert werden. Eine Pedalplatte 5 ist über Kugellager 6 drehbeweglich mit der Hohlachse 2 verbunden.

Mehrere Dehnungsmessstreifen 7 sind zwischen der Kontermutter 4 und der drehbeweglich angeordneten Pedalplatte 5 auf der Hohlachse 2 angebracht. Die Dehnungsmessstreifen 7 sind so angeordnet und miteinander verschaltet, dass zwei Wheatstone-Messbrücken gebildet werden, die eine getrennte Messung der Kräfte ermöglichen, die über das Pedal 1 auf die Tretkurbel in tangentialer bzw. radialer Richtung relativ zu den Tretkurbelarmen übertragen werden. Die verwendeten Dehnungsmessstreifen 7 sind hinsichtlich der Biegespannungsmessung optimiert, da im wesentlichen die Biegeverformung der Hohlachse 2 in dem von den Dehnungsmessstreifen 7 erfassten Bereich für die Bestimmung der an der Tretkurbel angreifenden Kräfte verwendet werden. Die Dehnungsmessstreifen 7 sind durch eine Abdeckung 8 vor Beschädigung oder Verschmutzung geschützt.

In dem Zwischenraum zwischen der drehbeweglich angeordneten Pedalplatte 5 und der Hohlachse 2 ist auf der Hohlachse 2 eine Leiterplatte 9 befestigt, auf der die einzelnen Bauteile der Verstärkerelektronik 10 angeordnet und miteinander verbunden sind. Ebenfalls auf der Leiterplatte 9 angeordnet befindet sich ein Lautsprecher 11 zur Erzeugung der akustischen Signale.

Im Inneren der Hohlachse 2 befindet sich eine Batterie 12, die mit der Verstärkerelektronik 10 elektrisch leitend verbunden ist und die Messvorrichtung, die Verstärkerelektronik 10 und den Lautsprecher 11 mit Energie versorgt. Die Batterie 12 kann aus der Hohlachse 2 entnommen und ausgewechselt werden.

In Fig. 2 ist der prinzipielle Aufbau einer Verstärkerelektronik 10 dargestellt. Zwei Wheatstone-Messbrücken 13, 14 sind über geeignete Verstärker 15 und Gleichrichter 16 mit einem Frequenzgenerator 17 verbunden, der den damit verbundenen Lautsprecher 11 ansteuert. Als Frequenzgenerator 17 eignet sich jedes elektronische Schaltungselement, welches zur Frequenzerzeugung für einen akustischen Signalgeber verwendet werden kann.

Während bei der Wheatstone-Messbrücke 14, mit welcher die radial auf die Tretkurbel wirkenden Kräfte gemessen werden, die verstärkten Messsignale gleichgerichtet und dem Frequenzgenerator 17 zugeführt werden, werden von der Wheatstone-Messbrücke 13, welche die tangential auf den Tretkurbelarm drückenden Kräfte erfasst, nur diejenigen Signale dem Frequenzgenerator 17 zugeführt, die tangential entgegen der Umdrehungsrichtung an dem Tretkurbelarm angreifen. Eine derartige Unterscheidung kann bei geeigneter Anordnung der Dehnungsmessstreifen 7 dadurch herbeigeführt werden, dass beispielsweise nur gemessene Zugkräfte oder Druckkräfte gleichgerichtet und dem Frequenzgenerator 17 zugeführt werden.

In Fig. 3 ist schematisch der Aufbau einer komplexeren Verstärkerelektronik 18 dargestellt. Die Signale der Wheatstone-Messbrücken 13, 14 werden ebenfalls durch Verstärker 15 verstärkt, dann jedoch über Analog-Digital-Wandler 19 in digitale Eingangssignale für einen Mikrocomputer 20 gewandelt werden. Der Mikrocomputer 20 übernimmt hierbei Funktionen wie die Signalaufbereitung, die Frequenzaufbereitung sowie die Einschaltautomatik. Nach Verarbeitung der Eingangssignale wird ein digitales Ausgangssignal über einen Digital-Analog-Wandler 21 in ein Steuersignal zur Ansteuerung des Lautsprechers 11 gewandelt.

Der Mikrocomputer 20 integriert die Signalaufbereitung der Eingangssignale und einen Frequenzgenerator zur Ansteuerung des Lautsprechers 11. Neben der Ermittlung der gemessenen Kraftanteile, die akustisch angezeigt werden sollen, werden durch einen Bandfilter Störsignale mit einer Frequenz außerhalb etwa 0,5-3 Hz unterdrückt. Zudem kann die Ansprechempfindlichkeit der Verstärker 15 stufig oder kontinuierlich verändert werden, um den Schwellenwert der durch akustische Signale angezeigten gemessenen Kräfte vorgeben zu können.

Der Mikrocomputer 20 ist auch mit einem nicht dargestellten Einschaltsensor, beispielsweise einem Reed-Schalter, verbunden und schaltet die Verstärkerelektronik aus einem Energiesparmodus heraus ein, sobald der Einschaltsensor Impulse in einem vorgegebenen Frequenzbereich nachweist, die auf Rotationsbewegungen der Tretkurbel zurückzuführen sind. Dies kann beispielsweise durch einen an der Tretkurbel gegenüber dem Reed-Schalter angeordneten Magneten mit einfachen Mitteln realisiert werden.

In den dargestellten Ausführungsbeispielen ist die Kraftmessvorrichtung und die Auswertevorrichtung vollständig im Pedal 1 angeordnet. Es ist aber ebenfalls möglich und für bestimmte Anforderungen zweckmäßig, dass die Kraftmessvorrichtung an einem Tretkurbelarm angeordnet ist. In gleicher Weise kann vorgesehen sein, dass ein akustischer Signalgeber mit einer gegebenenfalls benötigten Messverstärkeranordnung an einem Pedal oder an dem Tretkurbelarm angeordnet ist. Es sind auch Ausführungen denkbar, bei denen die Auswertevorrichtung mit einem externen akustischen Signalgeber verbindbar ist, so dass beispielsweise Kopf- oder Ohrhörer per Funk von der Auswertevorrichtung angesteuert werden.

## Patentansprüche

1. Verfahren zur Auswertung der auf eine Tretkurbel eines Fahrrads oder Ergometers ausgeübten, gemessenen Kräfte, **dadurch gekennzeichnet, dass** die nicht in tangentialer Richtung im Umdrehungssinn angreifenden Kräfte ermittelt und akustisch signalisiert werden.

2. Verfahren nach Anspruch 1, **dadurch gekennzeichnet, dass** eine betragsmäßige Änderung der ermittelten Kräfte durch eine vorbestimmte Frequenzänderung des akustischen Signals angezeigt wird.

3. Verfahren nach Anspruch 1, **dadurch gekennzeichnet, dass** auch die in tangentialer Richtung im Umdrehungssinn angreifenden Kräfte und die Umdrehungsfrequenz ermittelt werden.

4. Verfahren nach Anspruch 1, **dadurch gekennzeichnet, dass** aus den ermittelten Kräften die dafür aufgewandte Leistung bestimmt und bei vorgebbaren Grenzwerten der Leistung ein akustisches Signal erzeugt wird.

5. Verfahren nach Anspruch 1, **dadurch gekennzeichnet, dass** die Messwerte der ermittelten Kräfte und/oder bestimmten Leistung in einem Speichermedium gespeichert werden.

6. Vorrichtung zur Durchführung des in Anspruch 1 angegebenen Verfahrens mit einer Kraftmessvorrichtung und einer damit in Verbindung stehenden Auswertevorrichtung, **dadurch gekennzeichnet, dass** mittels der Auswertevorrichtung der Anteil der nicht in tangentialer Richtung im Umdrehungssinn angreifenden Kräfte bestimmbar und ein davon abhängiges Signal als Steuerungssignal für einen akustischen Signalgeber (11) verwendbar ist.

7. Vorrichtung nach Anspruch 6, **dadurch gekennzeichnet, dass** die Kraftmessvorrichtung an einer Pedalachse (2) angeordnet ist.

8. Vorrichtung nach Anspruch 6, **dadurch gekennzeichnet, dass** die Kraftmessvorrichtung an einem Tretkurbelarm angeordnet ist.

9. Vorrichtung nach Anspruch 6, **dadurch gekennzeichnet, dass** die angreifenden Kräfte mittels Dehnungsmessstreifen (7) ermittelbar sind.

10. Vorrichtung nach Anspruch 6, **dadurch gekennzeichnet, dass** die Abhängigkeit der erzeugten akustischen Signale von den gemessenen Kräften einstellbar ist.

11. Vorrichtung nach Anspruch 6, **dadurch gekennzeichnet, dass** eine relative Änderung der gemessenen Kräfte durch eine vorbestimmte Frequenzänderung des akustischen Signals anzeigbar ist.

12. Vorrichtung nach Anspruch 6, **dadurch gekennzeichnet, dass** ein akustischer Signalgeber (11) mit einer gegebenenfalls benötigten Messverstärkeranordnung (10, 18) an einem Pedal (1) oder an dem Tretkurbelarm angeordnet ist.

13. Vorrichtung nach Anspruch 6, **dadurch gekennzeichnet, dass** die Auswertevorrichtung mit einem externen akustischen Signalgeber verbindbar ist.

14. Vorrichtung nach Anspruch 6, **dadurch gekennzeichnet, dass** die akustischen Signale mittels mit der Auswertevorrichtung in Verbindung stehenden Kopf- oder Ohrhörern erzeugbar sind.

15. Vorrichtung nach Anspruch 6, **dadurch gekennzeichnet, dass** an dem Pedal (1) ein Umdrehungssensor und ein Energiespeicher (12) angeordnet sind, die jeweils mit der Auswertevorrichtung in Verbindung stehen.

16. Vorrichtung nach Anspruch 6, **dadurch gekennzeichnet, dass** auch die in tangentialer Richtung im Umdrehungssinn angreifenden Kräfte und die Umdrehungsfrequenz ermittelbar sind.

17. Vorrichtung nach Anspruch 6, **dadurch gekennzeichnet, dass** bei vorgebbaren Grenzwerten der gemessenen Leistung ein akustisches Signal erzeugbar ist.

18. Vorrichtung nach Anspruch 6, **dadurch gekennzeichnet, dass** die Messwerte der gemessenen Kräfte und/oder Leistung in einem Speichermedium speicherbar sind.
